(19) Europäisches Patentamt
European Patent Office
Office européen des brevets

(11) Veröffentlichungsnummer: **0 329 013 A2**

# (12) EUROPÄISCHE PATENTANMELDUNG

(21) Anmeldenummer: **89102215.4**

(22) Anmeldetag: **09.02.89**

(51) Int. Cl.⁴: **C07D 233/64 , C07D 401/12 , C07D 409/14 , C07D 405/14 , A61K 31/415**

Patentansprüche für folgende Vertragsstaaten: ES + GR.

(30) Priorität: **16.02.88 DE 3804793**

(43) Veröffentlichungstag der Anmeldung: **23.08.89 Patentblatt 89/34**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Henning, Rainer, Dr.**
**Rotenhofstrasse 31**
**D-6234 Hattersheim am Main(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim (Taunus)(DE)**

(54) **Renin-hemmende Aminosäurederivate.**

(57) Die Erfindung betrifft Verbindungen der Formel I

$$A - B - NH - \underset{}{\overset{R^1}{CH}} - \underset{OR^9}{CH} - \overset{R^2}{CH} - R^3 \qquad (I),$$

worin A für einen substituierten Thio-, Sulfinyl- oder Sulfonyl-alkanoyl-Rest oder einen vier- bis elfgliedrigen Heterocyclus steht, B ein Aminosäurerest ist, $R^1$ Wasserstoff, (subst.)Alkyl, (subst.)Cycloalkyl oder Aryl bedeutet, $R^2$ für Wasserstoff, (subst.)Alkyl oder Aryl steht, $R^3$ einen Rest der Formel $-(CH_2)_q-(X)_r-(CH_2)_s\ R^6$ mit X = $CF_2$, CO oder $CHR^8$, $R^6$ = (subst.)Heteroaromat, $R^8$ = Alkyl, Alkoxy, Alkylthio, Alkylamino, Hydroxy, Azid oder Halogen, q und s = 1 bis 4 und r = 0 oder 1 bedeuten, und $R^9$ Wasserstoff, (subst.)-Alkyl, Alkanoyl, Cycloalkanoyl, (subst.)Aroyl oder (subst.)Aryl ist sowie deren Salze. Es werden außerdem Verfahren zur Herstellung dieser Verbindungen sowie ihre Verwendung als Renin-Inhibitoren beschrieben.

EP 0 329 013 A2

## Renin-hemmende Aminosäurederivate

Die Erfindung betrifft Aminosäurederivate, die die Wirkung des natürlichen Enzyms Renin hemmen sowie Verfahren zu ihrer Herstellung und ihre Verwendung.

Aus der europäischen Patentanmeldung Nr. 236 734 sind durch schwefelhaltige Gruppen substituierte 5-Amino-4-hydroxyvalerylderivate mit Renin-hemmender Wirkung bekannt.

Überraschenderweise wurde nun gefunden, daß Verbindungen, die sich vor allem in der Struktur der C-terminalen Bausteine von den in EP-A 236 734 beschriebenen Verbindungen unterscheiden, hochwirksame Renin-inhibitoren sowohl in vitro als auch in vivo sind.

Die Erfindung betrifft Verbindungen der Formel I

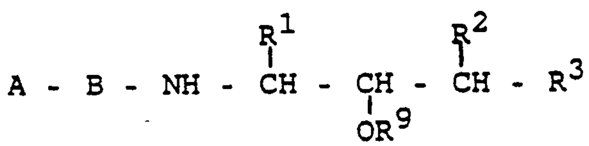

(I),

in welcher

A einen Rest der Formel II

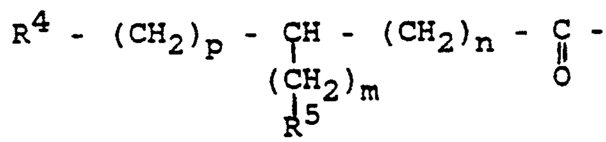

(II)

bedeutet, worin

$R^4$ a) für einen Rest der Formel IV

$R^7$-S(O)$_t$- (IV)

steht, mit

$R^7$ ($C_1$-$C_{10}$-Alkyl, welches durch eine oder mehrere funktionelle Gruppen, beispielsweise Oxo, Hydroxy, verethertes Hydroxy, z.B. ($C_1$-$C_6$)-Alkoxy, wie Methoxy oder Ethoxy, oder Phenyloxy; verestertes Hydroxy, z.B. ($C_1$-$C_6$)-Alkanoyloxy, wie Acetoxy; Halogen, z.B. Chlor oder Brom; Hydroxysulfonyloxy; Carboxy; verestertes Carboxy, z.B. ($C_1$-$C_4$)-Alkoxycarbonyl, wie Methoxy- oder Ethoxycarbonyl; amidiertes Carboxy, z.B. Carbamoyl oder Mono-oder Di-($C_1$-$C_4$)-alkylcarbamoyl, wie Methyl- oder Dimethylcarbamoyl; Cyano; Amino; substituiertes Amino, z.B. Mono-($C_1$-$C_4$)-alkylamino oder Di-($C_1$-$C_4$)-alkylamino; Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom - ist, substituiert sein kann;

($C_2$-$C_8$)-Alkenyl, ($C_2$-$C_8$)-Alkinyl, die beide wie Alkyl substituiert sein können;

Hydroxy; ($C_1$-$C_6$)-Alkoxy; ($C_3$-$C_8$)-Cycloalkyl; ($C_5$-$C_{10}$)-Bicycloalkyl; ($C_8$-$C_{10}$)-Tricycloalkyl; ($C_3$-$C_8$)-Cycloalkyl-($C_1$-$C_{10}$)-alkyl; ($C_6$-$C_{14}$)-Aryl, ($C_6$ $C_{14}$)-Aryloxy, ($C_6$-$C_{14}$)-Aryl-($C_1$-$C_{10}$) alkyl, die im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sind; einen gesättigten, aromatischen oder teilaromatischen Heterocyclus, der mono-, bi- oder tricyclisch sein kann und mindestens ein Kohlenstoffatom, ein bis vier Stickstoffatome und/oder ein Schwefel- oder Sauerstoffatom als Ringglieder enthält und durch einen, zwei oder drei, vorzugsweise einen oder zwei, gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino, Phenyl oder Trifluormethyl substituiert ist; Amino, substituiertes Amino, z.B. Mono-($C_1$-$C_4$)- alkylamino, Di-($C_1$-$C_4$)-alkylamino, Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom -ist, oder durch Oxo substituiert sein kann;

und

t 0, 1 oder 2; oder

b) einen gesättigten oder teilweise ungesättigten vier-bis zehngliedrigen monocyclischen oder sieben- bis elfgliedrigen bicyclischen Heterocyclus mit jeweils einem Schwefelatom oder einer SO- oder $SO_2$-Gruppe,

die durch einen oder zwei ($C_1$-$C_6$)-Alkyl-, ($C_6$-$C_{14}$)-Aryl-oder ($C_6$-$C_{14}$)-Aryl-($C_1$-$C_6$) Alkylreste substituiert sein können, wobei jeweils der Arylteil gegebenenfalls wie unter a) beschrieben substituiert sein kann, bedeutet;

$R^5$ Phenyl, 2- oder 3-Thienyl, 2-, 3- oder 4-Pyridyl, 1-, 2- oder 4-Imidazolyl, 1- oder 2-Naphthyl oder 2- oder 3-Benzo[b]thienyl bedeutet, die jeweils gegebenenfalls durch einen, zwei oder drei Reste aus der Gruppe Hydroxy, ($C_1$-$C_4$)-Alkyl, ($C_1$-$C_4$)-Alkoxy, Cl, F, Br, Nitro, Amino, Acetamido, Trifluormethyl oder einen Methylendioxy-Rest substituiert sind;

m 0, 1, 2 oder 3;

n 0, 1 oder 2;

p 0, 1 oder 2 bedeuten;

B einen N-terminal mit A und C-terminal mit

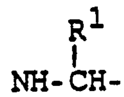

verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin, 1-, 3-oder 4-Pyrazolylalanin bedeutet;

$R^1$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl, ($C_6$-$C_{14}$)-Aryl-($C_1$-$C_4$)-alkyl oder 1,3-Dithiolan-2-yl-($C_1$-$C_4$)-alkyl bedeutet;

$R^2$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet;

$R^3$ einen Rest der Formel III

$-(CH_2)_q - (X)_r - (CH_2)_s - R^6$ (III) bedeutet, worin

$R^6$ für Wasserstoff, Hydroxy, Amino oder einen fünf-oder sechsgliedrigen monocyclischen oder neun-oder zehngliedrigen bicyclischen Heteroaromaten mit mindestens einem Kohlenstoffatom, einem bis vier Stickstoffatomen und/oder einem Schwefel-oder Sauerstoffatom als Ringglieder steht;

X $-CF_2-$, $-CO-$ oder $-CHR^8-$ bedeutet, wobei

$R^8$ für ($C_1$-$C_7$)-Alkyl, ($C_1$-$C_5$)-Alkoxy, ($C_1$-$C_5$)-Alkylthio, ($C_1$-$C_5$)-Alkylamino, -OH, $-N_3$, -F, -Cl, -Br oder -I steht;

q und s unabhängig voneinander 0, 1, 2, 3 oder 4 bedeuten; und

r 0 oder 1 bedeutet, mit der Maßgabe, daß falls $R^4$ für einen Rest der Formel $R^7$-$S(O)_t$- (IV) mit t = 2 steht, r = 0 ist oder n ≠ 0 (Formel II) ist; und

$R^9$ Wasserstoff; ($C_1$-$C_{10}$)-Alkyl; ($C_1$-$C_6$)-Alkanoyl; ($C_6$-$C_9$)-Cycloalkanoyl; Phenyl, Phenyl-($C_1$-$C_4$)-alkyl, Benzoyl, die jeweils gegebenenfalls im Aromaten durch einen, zwei oder drei Reste aus der Gruppe ($C_1$-$C_4$)-Alkyl, ($C_1$-$C_4$)-Alkoxy, Cl, F, Br, Nitro, Trifluormethyl oder ein Methylendioxy substituiert sind; ($C_1$-$C_6$)-Alkanoyloxy-($C_1$-$C_2$)-alkyl, ($C_1$-$C_6$)-Alkoxycarbonyloxy-($C_1$-$C_2$)-alkyl oder

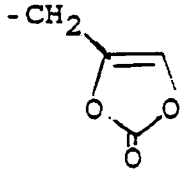 bedeutet,

sowie deren physiologisch verträgliche Salze.

Die Chiralitätszentren in den Verbindungen der Formel I können die R-, S- oder R,S-Konfiguration aufweisen.

Alkyl kann geradkettig oder verzweigt sein. Entsprechendes gilt für davon abgeleitete Rest, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

($C_6$-$C_{14}$)-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit ($C_1$ $C_6$)-Alkyl verknüpften unsubstituierten oder substituierten ($C_6$-$C_{14}$)-Aryl-Reste, wie z.B. Benzyl, α-und β-Naphthylmethyl, Halobenzyl und Alkoxybenzyl, wobei Aralkyl jedoch nicht auf die genannten Reste beschränkt ist.

Bicycloalkyl ist z.B. Bicyclohexyl, -heptyl, -octyl, -nonyl oder -decyl, z.B. Bicyclo[3.1.0]hex-1-, -2- oder -3-yl, Bicyclo[4.1.0]hept-1- oder -7-yl, Bicyclo[2.2.1]hept-2-yl, z.B. endo- oder exo-Norbornyl, Bicyclo[3.2.1]-oct-2-yl, Bicyclo[3.3.0]oct-3-yl oder Bicyclo[3.3.1]non-9-yl, ferner α- oder β-Decahydronaphthyl.

Tricycloalkyl enthält z.B. 8 bis 10 Kohlenstoffatome und ist z.B. Tricyclo[5.2.1.$0^{2,6}$]dec-8-yl oder Adamantyl, wie 1-Adamantyl. Ein fünf- oder sechsgliedriger monocyclischer oder neun- oder zehngliedriger bicyclischer Heteroaromat mit mindestens einem Kohlenstoffatom, ein bis vier Stickstoffatomen und/oder einem Schwefel- oder Sauerstoffatom als Ringglieder ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat dieser Reste. Dieser Heterocyclus kann an einem Stickstoffatom durch Oxido, ($C_1$-$C_6$)-Alkyl, z.B. Methyl oder Ethyl, Phenyl oder Phenyl-($C_1$-$C_4$)-alkyl, z.B. Benzyl, und/oder an einem oder mehreren Kohlenstoffatomen durch ($C_1$-$C_4$)-Alkyl, z.B. Methyl, Phenyl, Phenyl-($C_1$-$C_4$)-alkyl, z.B. Benzyl, Halogen, z.B. Chlor, Hydroxy, ($C_1$-$C_4$)-Alkoxy, z.B. Methoxy, Phenyl-($C_1$-$C_4$)-alkoxy, z.B. Benzyloxy, oder Oxo substituiert und teilweise gesättigt sein und ist beispielsweise 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-pyrimidinyl, 2-, 3- oder 5-Indolyl, substituiertes 2-Indolyl, z.B. 1-Methyl-, 5-Methyl-, 5-Methoxy, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Unter Alkanoyloxyalkyl versteht man beispielsweise Acetoxymethyl, Acetoxyethyl, Pivaloyloxymethyl, Pivaloyloxyethyl, 2,2-Dimethylbutyryloxymethyl, und unter Alkoxycarbonyloxyalkyl beispielsweise Ethoxycarbonyloxymethyl, Ethoxycarbonyloxyethyl, tert.-Butoxycarbonyloxymethyl sowie tert.-Butoxycarbonyloxyethyl.

Als S-, SO- oder $SO_2$-haltige Heterocyclen für $R^4$ kommen beispielsweise in Betracht: Dihydrothiophen, Tetrahydrothiophen, Thiopyran, Dihydrothiopyran, Tetrahydrothiopyran, Hexahydrothiepin, Octahydrothiocin, Octahydrocyclopenta[b]thiophen, Octahydrocyclopenta[c]thiophen, Dihydrobenzothiophen, Octahydrobenzothiophen, Octahydroisobenzothiophen, Dihydroisobenzothiophen, Decahydrocyclohepta[b]thiophen, Decahydrocyclohepta[c]thiophen sowie deren SO- und $SO_2$-Analoga.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in welcher

A einen Rest der Formel II bedeutet, worin

$R^4$ a) für einen Rest der Formel IV steht, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2-Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo[2.2.l]hept-2-yl, 1-Adamantyl, Cyclopropylmethyl, Cyclobutylmethyl, Cyclopentylmethyl oder Cyclohexylmethyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-,

3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, substituiertes Hydroxy, z.B. Methoxy, Ethoxy oder n-Butoxy, oder Aryloxy, z.B. Phenoxy 4-Hydroxyphenoxy oder 3,4-Methylendioxyphenoxy, Amino oder substituiertes Amino, z.B. Methylamino, Ethylamino, Isopropylamino, n- oder tert.-Butylamino, Dimethylamino oder Diethylamino, oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl; und

t 0, 1 oder 2; oder

b) einen gesättigten oder teilweise ungesättigten vier-bis siebengliedrigen monocyclischen oder sieben- bis zehngliedrigen bicyclischen Heterocyclus mit jeweils einem Schwefelatom oder einer SO- oder $SO_2$-Gruppe, wobei der monocyclische Heterocyclus durch ein oder zwei ($C_1$-$C_4$)-Alkyl- oder Phenylreste substituiert sein kann, bedeutet;

$R^5$ Phenyl, 2-Thienyl, 2-Pyridyl, 1-Naphthyl, 2-Benzo[b]thienyl oder 3-Benzo[b]thienyl, die jeweils gegebenenfalls durch einen, zwei oder drei Reste aus der Gruppe Methyl, Ethyl, Isopropyl, tert.-Butyl, Methoxy, Hydroxy, F, Cl, Nitro, Trifluormethyl oder einen Methylendioxyrest substituiert sind, bedeutet;

m 0, 1 oder 2 ist;

n 0 oder 1 ist;

p 0 oder 1 bedeutet;

B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-methyl-Histidin, Benzodioxol-5-yl-alanin, 1,2,3,4-Tetrahydroisochinolin-3-carbonsäure, Homophenylalanin, 2-Amino-4-(2-thienyl)buttersäure, (Z)-Dehydrophenylalanin, (E)-Dehydrophenylalanin, 1,3-Dioxolan-2-yl-alanin steht;

$R^1$ Isobutyl, Benzyl oder Cyclohexylmethyl bedeutet;

$R^2$ Wassertoff, Methyl, Isopropyl oder Isobutyl ist;

$R^3$ für einen Rest der Formel III steht; worin

$R^6$ Wasserstoff, Hydroxy, Amino oder einen fünf- oder sechsgliedrigen monocyclischen oder neun- oder zehngliedrigen bicyclischen Heteroaromaten mit mindestens einem Kohlenstoffatom, einem bis vier Stickstoffatomen und/oder einem Schwefel- oder Sauerstoffatom als Ringglieder bedeutet;

X $-CF_2-$, -CO- oder $-CHR^8-$ ist, wobei

$R^8$ ($C_1$-$C_4$)-Alkyl, beispielsweise Methyl, Ethyl, n-Propyl oder Isopropyl, ($C_1$-$C_4$)-Alkoxy, beispielsweise Methoxy, Ethoxy, n-Propoxy oder Isopropoxy, -OH, $-N_3$, -F oder -Cl bedeutet;

q und s unabhängig voneinander 0, 1, 2 oder 3 sind; r 0 oder 1 ist, mit der Maßgabe, daß falls $R^4$ für einen Rest der Formel IV mit $t = 0$ steht, $r = 0$ ist oder $n \neq 0$ (Formel II) ist;

und

$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;

sowie deren physiologisch verträgliche Salze.

Insbesondere sind bevorzugt Verbindungen der Formel I, in welcher

A für einen Rest der Formel II steht, worin

$R^4$ a) einen Rest der Formel IV bedeutet, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2-Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo[2.2.1]hept-2-yl, 1-Adamantyl, Cyclopropylmethyl, Cyclobutylmethyl, Cyclopentylmethyl oder Cyclohexylmethyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder a- oder β-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, substituiertes Hydroxy, z.B. Methoxy, Ethoxy oder n-Butoxy, oder Aryloxy, z.B. Phenoxy, 4-Hydroxyphenoxy oder 3,4 Methylendioxyphenoxy, Amino oder substituiertes Amino, z.B. Methylamino, Ethylamino, Isopropylamino, n- oder tert.-Butylamino, Dimethylamino oder Diethylamino, oder

Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl; und
t 0, 1 oder 2; oder b) für Dioxide von Tetrahydrothiophen, Tetrahydrothiopyran, Hexahydrothiepin, Octahydrocyclopenta[b]thiophen, Octahydrocyclopenta[c]thiophen, Octahydrobenzothiophen oder Octahydroisobenzothiophen steht;
$R^5$ Phenyl, 2-Thienyl, 2-Pyridyl, 1-Naphthyl, die jeweils gegebenenfalls durch Hydroxy, Dihydroxy, Methoxy, Dimethoxy, Ethoxy, Isopropoxy, F, Cl oder Methylendioxy substituiert sind, bedeutet;
m 1 oder 2 ist;
n 0 und
p 0 oder 1 bedeuten;
B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)buttersäure, (Z)-Dehydrophenylalanin, (E)-Dehydrophenylalanin, 1,3-Dioxolan-2-yl-alanin steht;
$R^1$ Isobutyl, Benzyl oder Cyclohexylmethyl bedeutet;
$R^2$ Wasserstoff, Methyl, Isopropyl oder Isobutyl ist;
$R^3$ für einen Rest der Formel III steht, worin
$R^6$ einen fünf- oder sechsgliedrigen monocyclischen oder neun- oder zehngliedrigen bicyclischen Heteroaromaten mit mindestens einem Kohlenstoffatom, einem oder zwei Stickstoffatomen und/oder einem Schwefel- oder Sauerstoffatom als Ringglieder bedeutet;
X $-CF_2-$, -CO- oder $-CHR^8-$ ist, wobei
$R^8$ Methyl, Ethyl, Isopropyl, Methoxy, Ethoxy, Isopropoxy, -OH, $-N_3$ oder -F bedeutet;
q und s unabhängig voneinander 1, 2 oder 3 sind;
r 0 oder 1 ist, mit der Maßgabe, daß falls $R^4$ für einen Rest der Formel IV mit t= 2 steht, r= 0 ist; und
$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;
sowie deren physiologisch verträgliche Salze.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln Va und Vb:

A-OH (Va) A-B-OH (Vb)

Fragmente einer Verbindung der Formel I mit einer endständigen Aminogruppe besitzen die nachstehenden Formeln VIa und VIb:

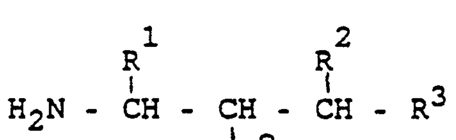 (VIa)

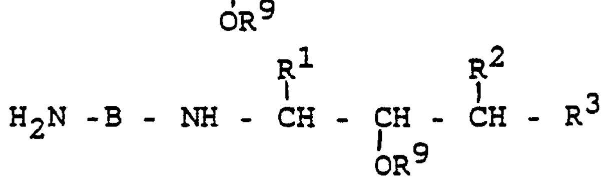 (VIb)

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:

Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel VII

$$H_2N - \overset{R^1}{\overset{|}{CH}} - \underset{OR^9}{\underset{|}{CH}} - \overset{R^2}{\overset{|}{CH}} - R^3 \qquad (VII)$$

worin $R^1$, $R^2$, $R^3$ und $R^9$ wie oben definiert sind, erfolgt ausgehend von optisch aktiven $\alpha$-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppe Aminoalkohole der Formel VII ($R^9$ = H) ergibt. Alternativ dazu werden in an sich bekannter Weise aus den geschützten Aminoaldehyden über die Allylamine die Epoxide hergestellt. Diese können entweder direkt mit den entsprechenden Arylalkyl-Nucleophilen umgesetzt werden, oder man öffnet das Epoxid zunächst mit Trimethylsilylchlorid und NaI in Acetonitril, spaltet den Silylether mit CsF und schützt das Iodid mit 2,2-Dimethoxypropan unter Säurekatalyse als Oxazolidin. Dieses Iodid kann mit weniger reaktiven Nucleophilen umgesetzt werden.

Zur Synthese der um eine $CH_2$-Gruppe verlängerten Arylalkyl-substituierten Aminoalkoholen geht man zum Beispiel von Boc-ACHPA-OEt (hergestellt nach J.Med.Chem. 28, 1779 (1985)) aus. Zunächst erfolgt N,O-Schützung, dann die Reduktion der Esterfunktion und schließlich die Umwandlung der Hydroxyfunktion in ein Bromid, das unter analogen Bedingungen wie die bereits genannten Elektrophile mit Arylalkyl-Nucleophilen umgesetzt werden kann. In Frage kommende Arylalkyl-Nucleophile sind z.B. Acetylimine und Acetylhydrazone. Weitere Verbindungen der Arylalkyl-substituierten Aminoalkohole mit verlängerter(n) $CH_2$-Gruppe(n) können nach den allgemein üblichen Methoden zur Kettenverlängerung erhalten werden.

Falls der gewählte Syntheseweg zu Diastereomeren bezüglich des $OR^9$ tragenden Zentrums führt, können diese in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et. al., J. org. Chem. 34, 2543 (1969)).

Die Herstellung N-geschützter Aminosäurealdehyde erfolgt nach B. Castro et al. (Synthesis 1983, 676).

Die Addition der Arylalkyl-Nucleophile an die genannten N-geschützten Elektrophile (bevorzugterweise N-tert.-Butoxycarbonyl- und Benzyloxycarbonyl-Schutzgruppen) erfolgt in einem gegenüber Basen inerten Lösungsmittel wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

In einer erhältlichen Verbindung der Formel I kann man eine Thiogruppe zu einer Sulfinyl- oder Sulfonylgruppe oder eine Sulfinylgruppe zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe oder Sulfinylgruppe selektiv in Gegenwart anderer funktioneller Gruppen der Verbindung der Formel I, z.B. der Amidfunktion und der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Säuren A oder B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf;

insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, Schweine-Renin) gemessen. Hierzu wird z.B. Humanplasma, welches sowohl Renin als auch Angiotensinogen enthält, bei 37° C mit der zu testenden Verbindung inkubiert. Anschließend wird die Konzentration des während der Inkubation gebildeten Angiotensin I mit einem Radioimmunoassay gemessen. Die in der vorliegenden Erfindung beschriebenen Verbindungen der allgemeinen Formel I zeigen in verwendeten in-vitro-Tests Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Zubereitungen, die diese Verbindung enthalten sowie ein Verfahren zu deren Herstellung. Bevorzugt ist die Anwendung bei Primaten, insbesondere beim Menschen.

Pharmazeutische Präparate enthalten eine wirksame Menge des Wirkstoffs der Formel I zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

Verzeichnis der verwendeten Abkürzungen:

ACHPA [3S,4S]-4-Amino-3-hydroxy-5-cyclohexylpentansäure
Boc tert.-Butoxycarbonyl

BuLi Butyl-Lithium
DC Dünnschichtchromatographie
DCC Dicyclohexylcarbodiimid
DCI Desorption Chemical Ionisation
DIP Diisopropylether
DNP 2,4-Dinitrophenyl
DME Dimethoxyethan
DMF Dimethylformamid
DOPA 3,4-Dihydroxyphenylalanin
EE Essigsäureethylester
EI Electron Impact
FAB Fast atom bombardment
H n-Hexan
HOBt 1-Hydroxybenzotriazol
M Molekularpeak
MeOH Methanol
MS Massenspektrum
MTB Methyl-tert.-butylether
NEM N-Ethylmorpholin
R.T. Raumtemperatur
Schmp. Schmelzpunkt
THF Tetrahydrofuran

Die sonstigen für Aminosäuren verwendeten Abkürzungen entsprechen dem in der Peptidchemie üblichen drei Buchstaben-Code wie er z.B. in Europ. J. Biochem. 138, 9-37 (1984) beschrieben ist. Falls nicht ausdrücklich anders angegeben, handelt es sich immer um Aminosäuren der L-Konfiguration.

Das nachstehende Beispiel dient der Erläuterung der vorliegenden Erfindung ohne diese darauf zu beschränken.

**Beispiel I**

**N-(2(R)-Benzyl-3-tert.-butylsulfonyl-propionyl)-His-1(S)-cyclohexyimethyl-2(S)-hydroxy-5-(2-pyridyl)-pentylamid**

und

**N-(2(S)-Benzyl-3-tert.-butylsulfonyl-propionyl)-His-1(S)-cyclohexylmethyl-2(S)-hydroxy-5-(2-pyridyl)-pentylamid**

240 mg N-2(R,S)-Benzyl-3-tert.-butylsulfonyl-propionyl)-His(DNP)-1(S)-cyclohexylmethyl-2(S)-hydroxy-5-(2-pyridyl)-pentylamid werden in 5 ml Acetonitril mit 100 mg Thiophenol 4 Stunden bei Raumtemperautr gerührt. Nach Einengen wird an Kieselgel mit Dichlormethan/Methanol (12:1), gesättigt mit konz. $NH_3$ chromatographiert.

Man erhält 50 mg des unpolaren (R)-Isomeren und 40 mg des polaren (S)-Isomeren.

(R)-Isomeres: $R_f$ 0,16 MS (FAB) : 680 (M+1)
(S)-Isomeres: $R_f$ 0,1 MS (FAB) : 680 (M+1)

a) **N-(2(R,S)-Benzyl-3-tert.-butylsulfonyl-propionyl)-His(DNP)-1(S)-cyclohexylmethyl-2(S)-hydroxy-5-(2-pyridyl)-pentylamid**

220 mg H-His(DNP)-1(S)-cyclohexylmethyl-2(S)-hydroxy-5-(2-pyridyl)-pentylamid werden mit 113 mg 2-(R,S)-Benzyl-3-tert.-butylsulfonyl-propionsäure, 82 mg DCC, 61 mg HOBt und 92 mg NEM in 4 ml DMF 20 Stunden bei Raumtemperatur gerührt. Nach Filtration wird mit EE verdünnt und je 1x mit 3 % $NaHCO_3$. 10 % Citronensäure, 3 % $NaHCO_3$ und gesättigter NaCl gewaschen, mit $Na_2SO_4$ getrocknet und eingeengt. Man erhält 240 mg der Titelverbindung als Harz (Isomerengemisch, $R_f$-Werte 0,6 und 0,55

($CH_2Cl_2$/MeOH = 10:1).

b) **H-His(DNP)-1(S)-cyclohexylmethyl-2(S)-hydroxy-5-(2-pyridyl)-pentylamid•Hydrochlorid**

230 mg Boc-His(DNP)-1(S)-cyclohexylmethyl-2(S)-hydroxy-5-(2-pyridyl)-pentylamid werden in 5 ml gesättigtem DME/HCl 90 Minuten gerührt, anschließend wird eingeengt.
Man erhält 220 mg der Titelverbindung als Harz. $R_f$ ($CH_2Cl_2$/MeOH = 10:1) 0,1.

c) **Boc-His(DNP)-1(S)-cyclohexylmethyl-2(S)-hydroxy-5-(2-pyridyl)-pentylamid**

Zu 1,9 g Boc-His(DNP)-OH, 360 µl Pyridin und 620 µl N-Ethylpiperidin in 50 ml $CH_2Cl_2$ werden bei -5°C 550 µl Pivaloylchlorid getropft. Nach 10 Minuten bei -5°C rührt man 10 Minuten bei +10°C. Nach erneutem Abkühlen auf -5°C gibt man 1,3 g 1(S)-Cyclohexylmethyl-2(S)-hydroxy-5-(2-pyridyl)-pentylamin (hergestellt aus 1,7 g 3-Boc-4(S)-cyclohexylmethyl-2,2-dimethyl-5-(3-(2-pyridyl)- propyl)-oxazolidin nach dem unter b) beschriebenen Verfahren und anschließender Freisetzung mit $Na_2CO_3$) in 20 ml $CH_2Cl_2$ zu. Nach 1 Stunde bei -5°C läßt man 16 Stunden bei Raumtemperatur stehen.
Man versetzt mit 50 ml gesättiger $Na_2CO_3$-Lösung und extrahiert 3mal mit EE. Die vereinigten organischen Extrakte werden über $Na_2SO_4$ getrocknet und eingeengt. Chromatographie an Kieselgel (EE/MeOH 10:1) liefert die Titelverbindung.
$R_f$ (EE/MeOH 10:1) = 0,25; MS (FAB) = 680 (M+1)

d) **3-Boc-4(S)-cyclohexylmethyl-2,2-dimethyl-5-(3-(2-pyridyl)-propyl)-oxazolidin**

367 µl 2-Picolin wird mit n-BuLi in wasserfreiem THF deprotoniert. Zur tiefroten Lösung wird bei -50°C 500 mg 3-Boc-4(S)-cyclohexylmethyl-2,2 dimethyl-5-(2-bromethyl)-oxazolidin in 10 ml THF zugegeben. Nach 15 Minuten wird $H_2O$ addiert und 3mal mit EE extrahiert. Nach dem Trocknen der organischen Phasen mit $Na_2SO_4$ wird eingeengt und an Kieselgel chromatographiert (Eluens: Toluol/EE 3:1)
$R_f$ (Toluol/EE 3:1) = 0,25; MS = 417 (M+1)

e) **3-Boc-4(S)-cyclohexylmethyl-2,2-dimethyl-5-(2-bromethyl)-oxazolidin**

Zu 690 mg 3-Boc-1(S)-cyclohexylmethyl-2,2-dimethyl-5-(2-hydroxyethyl)-oxazolidin, 2,6 g Triphenylphosphin und 1,6 g Pyridiniumbromid in 15 ml $CH_2Cl_2$ werden unter Argon 1,6 ml Azodicarbonsäurediethylester bei 20°C zugetropft. Nach 16 Stunden bei Raumtemperatur wird $H_2O$ zugegeben und mit 100 ml $CH_2Cl_2$ verdünnt. Die organische Phase wird 2mal mit gesättigter $NaHCO_3$-Lösung und 1mal mit gesättiger NaCl-Lösung gewaschen. Die mit $Na_2SO_4$ getrocknete organische Phase wird eingeengt, der Rückstand in wenig EE aufgenommen und zur Abtrennung von $PPh_3$ filtriert. Reinigung an Kieselgel liefert die Titelverbindung (Eluens: H/EE 15:1).
$R_f$ (H/EE 15:1) = 0,3; MS 404 (M)

f) **3-Boc-4(S)-cyclohexylmethyl-2,2-dimethyl-5-(2-hydroxyethyl)-oxazolidin**

10 g Boc-ACHPA-OEt, 500 mg p-Toluolsulfonsäure und 7,2 ml Dimethoxypropan werden in 160 ml Toluol unter Argon 2 Stunden auf 80°C erhitzt. Anschließend wird eingeengt. Der Rückstand wird bei 0°C zu einer Suspension aus 2 g $LiAlH_4$ in 200 ml THF zugetropft. Nach 2,5 Stunden bei 0°C werden 100 ml 5 %ige $NaHSO_4$-Lösung zugegeben und 3mal mit EE extrahiert. Die vereinigten organischen Phasen werden 1mal mit gesättiger $NaHCO_3$-Lösung gewaschen. Nach dem Trocknen mit $Na_2SO_4$ wird eingeengt und chromatographiert. (Eluens: H/EE 2:1).
$R_f$ (H/EE 4:1) = 0,1; MS = 342 (M+1)

**Beispiel 2**

**N-(2(R)-Benzyl-3-Cyclohexylsulfonyl-propionyl)-His-1(S)-cyclohexylmethyl-2(S)-hydroxy-5-(2-pyridyl)-pentylamid**

und

**N-(2(S)-Benzyl-3-Cyclohexylsulfonyl-propionyl)-His-1(S)-cyclohexylmethyl-2(S)-hydroxy-5-(2-pyridyl)-pentylamid**

270 mg N-2(R,S)-Benzyl-3-cyclohexylsulfonyl-propionyl)-His(DNP)-1-(S)-cyclohexylmethyl)-2(S)-hydroxy-5-(2-pyridyl) pentylamid werden in 3 ml Acetonitril mit 100 mg Thiophenol 2 Stunden gerührt. Nach Einengen wird an Kieselgel mit Dichlormethan/Methanol/konz. $NH_3$ (10:1:0,1) chromatographiert.

Man erhält:

80 mg unpolares (R)-Isomer (HPLC: $t_R$ 11,0 min (Nucleosil $C_{18}$ (7 $\mu$)), Laufmittel $CH_3CN/H_2O$ 34:66 (0,1 % TFA) 1,5 ml/min). und

40 mg polares (S)-Isomer, Schmp. 158-161 °C (HPLC: $t_R$ = 14,5 min, gleiche Bedingungen wie bei (R)-Isomeren).

**a) N-2(R,S)-Benyzl-3-cyclohexylsulfonyl-propionyl-His(DNP)-1-(S)-cyclohexylmethyl-2(S)-hydroxy-5-(2-pyridyl)-pentylamid**

Analog der in Beispiel 1a) angegebenen Vorschrift werden 180 mg H-His-(DNA)-1(S)-cyclohexylmethyl-2(S)-hydroxy-5-(2-pyridyl)-pentylamid mit 60 mg 2(R,S)-Benzyl-3-cyclohexylsulfonyl-propionsäure umgesetzt. Man erhält 280 mg der Titelverbindung als gelbliches Harz.

$R_f(SiO_2; CH_2Cl_2/MeOH\ 10:1) = 0,5$

**b) 2-(R,S)-Benzyl-3-cyclohexylsulfonyl-propionsäure**

3,2 g 2-(R,S)-Benzyl-3-cyclohexylsulfonyl-propionsäureethylester werden in 50 ml 5 N HCl 8 Stunden am Rückfluß gekocht. Die Mischung wird mit Dichlormethan extrahiert und der Extrakt mit $Na_2SO_4$ getrocknet. Nach Einengen erhält man 1,1 g der Titelverbindung als Öl.

**c) 2-(R,S)-Benzyl-3-cyclohexylsulfonyl-propionsäureethylester**

4,1 g 2-(R,S)-Benzyl-3-cyclohexylthio-propionsäureethylester werden in 50 ml trockene Dichlormethan mit 6,9 g 3-Chlorperbenzoesäure versetzt. Nach 3 Stunden bei R.T. werden nochmals 4 g 3-Chlorperbenzoesäure zugegeben. Nach weiteren 4 Stunden wird abgesaugt, je 1 x mit gesättigter $NaHSO_4$-Lösung und $Na_2CO_3$-Lösung gewaschen, mit $MgSO_4$ getrocknet und eingeengt. Man erhält 3,2 g der Titelverbindung als Öl

$R_f$ ($SiO_2$, Ethylacetat/Cyclohexan (1:1) = 0,55

**d) 2-(R,S)-Benzyl-3-cyclohexylthio-propionsäureethylester**

3 g 2-Benzylacrylsäureethylester werden zusammen mit 1,8 g Cyclohexylmercaptan und 0,2 g Natriumhydrid in 50 ml trockenen Ethanol gelöst. Nach 48 Stunden bei Raumtemperatur wird mit 2 N HCl angesäuert und mit Ether extrahiert. Man erhält 4,1 g der Titelverbindung als Öl.

$R_f$ ($SiO_2$; Ethylacetat/Cyclohexan 1:4) = 0,16

**e) 2-Benzylacrylsäure-ethylester**

127,6 g 2-Benzyl-malonsäurediethylester werden mit 20,4 g NaOH in 1 l trockenen Ethanol am Rückfluß gekocht. Nach 8 Stunden wird das Lösungsmittel entfernt, der Rückstand in 2 N HCl aufgenommen und mit Ether extrahiert. Die organische Phase wird getrocknet und eingeengt. Der Rückstand wird unter Kühlung

mit 51,2 ml Diethylamin und 43,7 ml 36 %iger Formaldehyd-Lösung versetzt. Nach Stehen über Nacht wird mit Ether extrahiert, getrocknet und eingeengt. Das Produkt wird destilliert.
Schmp. 70° C (0,01 mm); Ausbeute 56 g

**Beispiel 3**

**N-(2(R)-Benzyl-3-phenylsulfonyl-propionyl)-His-1(S)-cyclohexylmethyl-2(S)-hydroxy-5-(2-pyridyl)-pentylamid**

und

**N-(2(S)-Benzyl-3-phenylsulfonyl-propionyl)-His-1(S)-cyclohexylmethyl-2(S)-hydroxy-5-(2-pyridyl)-pentylamid**

320 mg N-(2(R,S)-Benzyl-3-phenylsulfonyl-propionyl)-His(DNP)-1(S)-cyclohexylmethyl-2(S)-hydroxy-5-(2-pyridyl)-pentylamid werden mit 120 mg Thiophenol in 3 ml Acetonitril 2 Stunden gerührt. Chromatographie an Kieselgel mit Dichlormethan/Methanol/konz. $NH_3$ (10 1:0,1) ergibt 50 mg (R)-Isomeres und 60 mg (S)-Isomeres
(R)-Isomeres: $t_R$(HPLC, Bedingungen wie Beispiel 2) : 8,5 min.
(S)-Isomeres: $t_R$ = 11,8 min.

**a) N-(2(R,S)-Benzyl-3-phenylsulfonyl-propionyl)-His(DNP)-1(S)-cyclohexylmethyl-2(S)-hydroxy-5-(2-pyridyl)-pentylamid**

Analog der in Beispiel 1a) angegebenen Vorschrift werden 180 mg H-His(DNP)-1(S)-cyclohexylmethyl-2(S)-hydroxy-5-(2-pyridyl)-pentylamid mit 60 mg 2(R,S)-Benzyl-3-phenylsulfonyl-propionsäure umgesetzt. Man erhält 320 mg der Titelverbindung.
$R_f$ ($SiCH_2$, $CH_2Cl_2$/MeOH 10:1) = 0,45.

**b) 2-(R,S)-Benzyl-3-phenylsulfony-propionsäure**

Hergestellt aus Thiophenol und 2-Benzylacrylsäureethylester analog den in Beispiel 2b-d, angegebenen Vorschriften.
$R_f$ ($SiO_2$; $CH_2Cl_2$/MeOH 10:1) = 0,3.

**Beispiel 4**

**N-[2-(R)-(4-Methoxy-benzyl)-3-tert.butylsulfonyl-propionyl]-His-1(S)-cyclohexylmethyl-2(S)-hydroxy-5-(2-pyyridyl)-pentylamid**

und

**N-[2-(S)-(4-Methoxy-benzyl)-3-tert.butylsulfony-propionyl]-His-1(S)-cyclohexylmethyl-2(S)-hydroxy-5-(2-pyridyl)-pentylamid**

94 mg N-[2 (R,S)-(4-methoxy-benzyl)-3-tert.butylsulfonyl-propionyl]-His(DNP)-1-(S)-cyclohexylmethyl-2-(S)-hydroxy-5-(2-pyridyl)-pentylamid werden in 4 ml Acetonitril mit 37 µl Thiophenol 2 Stunden gerührt. Nach Einengen wird an Kieselgel chromatographiert (Laufmittel: $CH_2Cl_2$/MeOH (0:1). Man erhält 22 mg (R)-Isomeres und 29 mg (S)-Isomeres.
(R)-Isomeres: $R_f$ 0,17 (Ethylacetat/MeOH = 10:1); MS(FAB): 710 (M+1)
(S)-Isomeres: $R_f$ 0,11 (Ethylacetat/MeOH = 10:1); MS(FAB): 710 (M+1).

**a) N-[2-(R,S)-(4-Methoxybenzyl)-3-tert.butylsulfonyl-propionyl]-His(DNP)-1-(S)-cyclohexylmethyl-2-(S)-hydroxy-5-(2-pyridyl)-pentylamid**

Analog der in Beispiel 1a) angegebenen Vorschrift werden 130 mg H-His(DNP)-1(S)-cyclohexylmethyl-2(S)-hydroxy-5-(2-pyridyl)-pentylamid mit 60 mg 2-(R,S)-(4-Methoxybenzyl)-3-tert.butylsulfonyl-propionsäure umgesetzt. Man erhält 94 mg der Titelverbindung als Isomerengemisch.
$R_f$ ($SiO_2$; $CH_2Cl_2$/MeOH = 10:1) 0,43 und 0,36.

**b) 2-(R,S)-(4-Methoxybenzyl)-3-tert.butylsulfonyl-propionsäure**

Hergestellt aus tert.Butylmercaptan und 2-(4-Methoxybenzyl)-malonsäure-diethylester analog den in Beispiel 2b-e angegebenen Vorschriften.
$R_f$ ($SiO_2$; $CH_2Cl_2$/MeOH 10:2) = 0,22.

**Beispiel 5**

**N-[2-(R,S)-(2-Thienyl-methyl)-3-tert.butylsulfonyl-propionyl]-His-1-(S)-cyclohexylmethyl-2-(S)-hydroxy-5-(2-pyridyl)-pentylamid**

Hergestellt analog Beispiel 1 aus N-[2-(R,S)-(2-Thienyl- methyl)-3-tert.butylsulfonyl-propionyl]-His(DNP)-1-(S)-cyclohexylmethyl-2-(S)-hydroxy-5-(2-pyridyl)-pentylamid und Thiophenol.
$R_f$ ($SiO_2$; Ethylacetat/Methanol 5:1) = 0,1
MS(FAB) = 686 (M+1)

**a) N-[2-(R,S)-(2-Thienylmethyl)-3-tert.butylsulfonyl-propionyl]-His(DNP)-1-(S)-cyclohexyl-2-(S)-hydroxy-5-(2-pyridyl)-pentylamid**

Hergestellt analog der in Beispiel la) angegebenen Vorschrift aus H-His(DNP)-1-(S)-cyclohexylmethyl-2-(S)-hydroxy-5-(2-pyridyl)-pentylamid und (R,S)-2-(2-Thienylmethyl)-3-tert.butylsulfonylpropionsäure.

**b) (R,S)-2-(2-Thienylmethyl)-3-tert.butylsulfonylpropionsäure**

Hergestellt aus 2-(2-Thienyl-methyl)-malonsäurediethylester und tert.Butylmercaptan analog den in Beispiel 2b-e angegebenen Vorschriften.

**Beispiel 6**

**N-[2-(R)-Benzyl-3-(2-furyl-methylsulfonyl)-propionyl]-His-1(S)-cyclohexylmethyl-2(S)-hydroxy-5-(2-pyridyl)-pentylamid**

und

**N-[2-(S)-Benzyl-3-(2-furylmethylsulfonyl)-propionyl]-His-1(S)-cyclohexylmethyl-2-(S)-hydroxy-5-(2-pyridyl)-pentylamid**

Hergestellt analog der in Beispiel 1 angegebenen Vorschrift aus N-[2-(R,S)-Benzyl-3-(2-furylmethylsulfonyl)-propionyl]-His(DNP)-1(S)-cyclohexylmethyl-2(S)-hydroxy-5-(2-pyridyl)-pentylamid und Thiophenol.
(R)-Isomer: $R_f$ ($SiO_2$; Methanol/Ethylacetat 1:5) = 0,25 MS(FAB): 704 (M+1)
(S)-Isomer: $R_f$ ($SiO_2$; Methanol/Ethylacetat 1:5) = 0,15 MS(FAB): 704 (M+1)

**a) N-[2-(R,S)-Benzyl-3-(2-furylmethylsulfonyl)-propionyl]-His(DNP)-1-(S)-cyclohexylmethyl-2-(S)-hydroxy-5-(2-pyridyl)-pentylamid**

Hergestellt analog der in Beispiel 1a) angegebenen Vorschrift aus H-His(DNP)-1(S)-cyclohexylmethyl-2-(S)-hydroxy-5-(2-pyridyl)-pentylamid und (R,S)-2-Benzyl-3-(2-furylmethylsulfonyl)-propionsäure

**b) (R,S)-2-Benzyl-3-(2-furylmethylsulfonyl)-propionsäure**

Hergestellt analog der in Beispiel 2b-d angegebenen Vorschriften aus 2-Benzylacrylsäureethylester und 2-Furylmethylmercaptan.

**Ansprüche**

1. Verbindung der Formel I

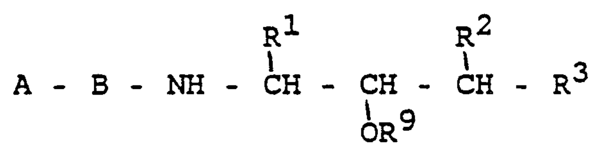 (I),

in welcher
A einen Rest der Formel II

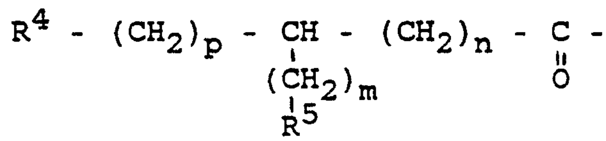 (II)

bedeutet, worin $R^4$ a) für einen Rest der Formel IV
$R^7\ S(O)_t$-     (IV),
steht, mit
$R^7$ ($C_1$-$C_{10}$-Alkyl, welches durch eine oder mehrere funktionelle Gruppen, beispielsweise Oxo, Hydroxy, ($C_1$-$C_6$)-Alkoxy, Phenyloxy, ($C_1$-$C_6$)-Alkanoyloxy, Halogen, Hydroxysulfonyloxy, Carboxy, ($C_1$-$C_4$)-Alkoxycarbonyl, Carbamoyl, Mono- oder Di-($C_1$-$C_4$)-alkylcarbamoyl, Cyano, Amino, Mono-($C_1$-$C_4$)-alkylamino, Di-($C_1$-$C_4$)-alkylamino, Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom -ist, substituiert sein kann;
($C_2$-$C_8$)-Alkenyl, ($C_2$-$C_8$)-Alkinyl, die beide wie Alkyl substituiert sein können;
Hydroxy; ($C_1$-$C_6$)-Alkoxy; ($C_3$-$C_8$)-Cycloalkyl; ($C_5$-$C_{10}$-Bicycloalkyl; ($C_8$-$C_{10}$)-Tricycloalkyl; ($C_3$-$C_8$)-Cycloalkyl-($C_1$-$C_{10}$)-alkyl; ($C_6$-$C_{14}$)-Aryl, ($C_6$-$C_{14}$)-Aryloxy, ($C_6$-$C_{14}$)-Aryl-($C_{1}$-$_{10}$)-alkyl, die im Arylteil gegebenenfalls durch einen oder zwei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sind; einen gesättigten, aromatischen oder teilaromatischen Heterocyclus, der mono-, bi- oder tricyclisch sein kann und mindestens ein Kohlenstoffatom, ein bis vier Stickstoffatome und/oder ein Schwefel- oder Sauerstoffatom als Ringglieder enthält und durch einen, zwei oder drei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino, Phenyl oder Trifluormethyl substituiert ist; Amino, substituiertes Amino, z.B. Mono-($C_1$-$C_4$)-alkylamino, Di-($C_1$-$C_4$)-alkylamino, Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom -ist, substituiert sein kann;
und
t 0, 1 oder 2; oder
b) einen gesättigten oder teilweise ungesättigten vier-bis zehngliedrigen monocyclischen oder sieben- bis elfgliedrigen bicyclischen Heterocyclus mit jeweils einem Schwefelatom oder einer SO- oder $SO_2$-Gruppe,

die durch einen oder zwei ($C_1$-$C_6$)-Alkyl-, ($C_6$-$C_{14}$)-Aryl-oder ($C_6$-$C_{14}$)-Aryl-($C_1$-$C_6$)-Alkylreste substituiert sein können, wobei jeweils der Arylteil gegebenenfalls wie unter a) beschrieben substituiert sein kann, bedeutet;

$R^5$ Phenyl, 2- oder 3-Thienyl, 2-, 3- oder 4-Pyridyl, 1-, 2- oder 4-Imidazolyl, 1- oder 2-Naphthyl oder 2- oder 3-Benzo[b]thienyl bedeutet, die jeweils gegebenenfalls durch einen, zwei oder drei Reste aus der Gruppe Hydroxy, ($C_1$-$C_4$)-Alkyl, ($C_1$-$C_4$)-Alkoxy, Cl, F, Br, Nitro, Amino, Acetamido, Trifluormethyl oder einen Methylendioxy-Rest substituiert sind;

m 0, 1, 2 oder 3;

n 0, 1 oder 2;

p 0, 1 oder 2 bedeuten;

B einen N-terminal mit A und C-terminal mit

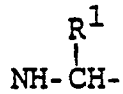

verknüpften Rest einer Aminosäure aus der Reihe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, $\beta$-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, $\beta$-2-Benzo[b]thienylalanin, $\beta$-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin, 1-, 3-oder 4-Pyrazolylalanin bedeutet;

$R^1$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$}-Aryl, ($C_6$-$C_{14}$)-Aryl ($C_1$-$C_4$)-alkyl oder 1,3-Dithiolan-2-yl-($C_1$-$C_4$)-alkyl bedeutet;

$R^2$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet;

R3 einen Rest der Formel III

$-(CH_2)_q - (X)_r - (CH_2)_s - R^6$ (III)

bedeutet, worin

$R^6$ für Wasserstoff, Hydroxy, Amino oder einen fünf-oder sechsgliedrigen monocyclischen oder neun-oder zehngliedrigen bicyclischen Heteroaromaten mit mindestens einem Kohlenstoffatom, einem bis vier Stickstoffatomen und/oder einem Schwefel-oder Sauerstoffatom als Ringglieder steht;

X $-CF_2-$, -CO- oder $-CHR^8-$ bedeutet, wobei

$R^8$ für ($C_1$-$C_7$)-Alkyl, ($C_1$-$C_5$)-Alkoxy, ($C_1$-$C_5$)-Alkylthio, ($C_1$-$C_5$)-Alkylamino, -OH, $-N_3$, -F, -Cl, -Br oder -I steht;

q und s unabhängig voneinander 0, 1, 2, 3 oder 4 bedeuten; und

r 0 oder 1 bedeutet, mit der Maßgabe, daß falls $R^4$ für einen Rest der Formel $R^7$ $S(O)_t$- (IV) mit t= 2 steht, r = 0 ist oder n $\neq$ 0 (Formel II) ist; und

$R^9$ Wasserstoff; ($C_1$-$C_{10}$)-Alkyl; ($C_1$-$C_6$)-Alkanoyl; ($C_6$-$C_9$,-Cycloalkanoyl; Phenyl, Phenyl-($C_1$-$C_4$)-alkyl, Benzoyl, die jeweils gegebenenfalls im Aromaten durch einen, zwei oder drei Reste aus der Gruppe ($C_1$-$C_4$)-Alkyl, ($C_1$-$C_4$)-Alkoxy, Cl, F, Br, Nitro, Trifluormethyl oder ein Methylendioxy substituiert sind; ($C_1$-$C_6$)-Alkanoyloxy-($C_1$-$C_2$)-alkyl, ($C_1$-$C_6$)-Alkoxycarbonyloxy-($C_1$-$C_2$)-alkyl oder

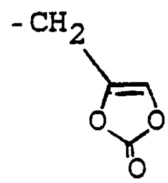

bedeutet,

sowie deren physiologisch verträgliche Salze.

2. Verbindung der Formel I, gemäß Anspruch 1, in welcher

A einen Rest der Formel II bedeutet, worin

$R^4$ a) für einen Rest der Formel IV steht, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2-Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo-[2.2.l]hept-2-yl, 1-Adamantyl, Cyclopropylmethyl, Cyclobutylmethyl, Cyclopentylmethyl oder Cyclohexylmethyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder $\alpha$- oder $\beta$-Naphthylmethyl, 2- oder 3-Pyrrolyl, 2-Furyl, 2-Thienyl, 2- oder 4-Imidazolyl, l-Methyl-2-, -4- oder -5-Imidazolyl, 1,3-Thiazol-2-yl, 2-, 3- oder 4-pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, Methoxy, Ethoxy oder n-Butoxy, Phenoxy, 4-Hydroxyphenoxy oder 3,4-Methylendioxyphenoxy, Amino oder Methylamino, Ethylamino, Isopropylamino, n- oder tert.-Butylamino, Dimethylamino oder Diethylamino, l-Pyrrolidinyl, 1-Piperidinyl oder 4-Morpholinyl; und

t 0, 1 oder 2; oder

b) einen gesättigten oder teilweise ungesättigten vier-bis siebengliedrigen monocyclischen oder sieben- bis zehngliedrigen bicyclischen Heterocyclus mit jeweils einem Schwefelatom oder einer SO- oder $SO_2$-Gruppe, wobei der monocyclische Heterocyclus durch ein oder zwei $(C_1\text{-}C_4)$-Alkyl- oder Phenylreste substituiert sein kann, bedeutet;

$R^5$ Phenyl, 2-Thienyl, 2-Pyridyl, 1-Naphthyl, 2-Benzo[b]thienyl oder 3-Benzo[b]thienyl, die jeweils gegebenenfalls durch einen, zwei oder drei Reste aus der Gruppe Methyl, Ethyl, Isopropyl, tert. Butyl, Methoxy, Hydroxy, F, Cl, Nitro Trifluormethyl oder einen Methylendioxyrest substituiert sind, bedeutet;

m 0, 1 oder 2 ist;

n 0 oder 1 ist;

p 0 oder 1 bedeutet;

B für einen bivalenten Rest aus der Gruppe Phenylalanin, Histidin, Tyrosin, Tryptophan, Methionin, Leucin, Isoleucin, Asparagin, Asparaginsäure, $\beta$-2-Thienylalanin, $\beta$-3-Thienylalanin, $\beta$-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, 1,3-Dioxolan-2-yl-alanin steht;

$R^1$ Isobutyl, Benzyl oder Cyclohexylmethyl bedeutet;

$R^2$ Wassertoff, Methyl, Isopropyl oder Isobutyl ist;

$R^3$ für einen Rest der Formel III steht; worin

$R^6$ Wasserstoff, Hydroxy, Amino oder einen fünf- oder sechsgliedrigen monocyclischen oder neun- oder zehngliedrigen bicyclischen Heteroaromaten mit mindestens einem Kohlenstoffatom, einem bis vier Stickstoffatomen und/oder einem Schwefel- oder Sauerstoffatom als Ringglieder bedeutet;

X $-CF_2$, -CO- oder $-CHR^8$- ist, wobei

$R^8$ $(C_1\text{-}C_4)$-Alkyl, $(C_1\text{-}C_4)$-Alkoxy, OH, $-N_3$, -F oder -Cl bedeutet;

q und s unabhängig voneinander 0, 1, 2 oder 3 sind;

r 0 oder 1 ist, mit der Maßgabe, daß falls $R^4$ für einen Rest der Formel IV mit $t = 0$ steht, $r = 0$ ist oder $n \neq 0$ (Formel II) ist;

und

$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;

sowie deren physiologisch verträgliche Salze.

3. Verbindung der Formel I, gemäß einem oder mehreren der Ansprüche 1 bis 2, in welcher

A für einen Rest der Formel II steht, worin

$R^4$ a) einen Rest der Formel IV bedeutet, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2-Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo-[2.2.1]hept-2-yl, 1-Adamantyl, Cyclopropylmethyl, Cyclobutylmethyl, Cyclopentylmethyl oder Cyclohexylmethyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, 2- oder 3-Pyrrolyl, 2-Furyl, 2-Thienyl, 2- oder 4-Imidazolyl, 1-Methyl 2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, Methoxy, Ethoxy oder n-Butoxy, Phenoxy, 4-Hydroxyphenoxy oder 3,4-Methylendioxyphenoxy, Amino oder Methylamino, Ethylamino, Isopropylamino, n- oder tert.-Butylamino, Dimethylamino oder 1-Pyrrolidinyl, 1-Piperidinyl oder 4-Morpholinyl; und

t 0, 1 oder 2; oder

b) für Dioxide von Tetrahydrothiophen, Tetrahydrothiopyran, Hexahydrothiepin, Octahydrocyclopenta[b]-thiophen, Octahydrocyclopenta[c]thiophen, Octahydrobenzothiophen oder Octahydroisobenzothiophen steht;

$R^5$ Phenyl, 2-Thienyl, 2-Pyridyl, 1-Naphthyl, die jeweils gegebenenfalls durch Hydroxy, Dihydroxy, Methoxy, Dimethoxy, Ethoxy, Isopropoxy, F, Cl oder Methylendioxy substituiert sind, bedeutet;

m 1 oder 2 ist;

n 0 und

p 0 oder 1 bedeuten;

B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin 2, 4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, l,3-Dioxolan-2-yl-alanin steht;

$R^1$ Isobutyl, Benzyl oder Cyclohexylmethyl bedeutet;

$R^2$ Wasserstoff, Methyl, Isopropyl oder Isobutyl ist;

$R^3$ für einen Rest der Formel III steht, worin

$R^6$ einen fünf- oder sechsgliedrigen monocyclischen oder neun- oder zehngliedrigen bicyclischen Heteroaromaten mit mindestens einem Kohlenstoffatom, einem oder zwei Stickstoffatomen und/oder einem Schwefel- oder Sauerstoffatom als Ringglieder bedeutet;

X $-CF_2-$, -CO- oder $-CHR^8-$ ist, wobei

$R^8$ Methyl, Ethyl, Isopropyl, Methoxy, Ethoxy, Isopropoxy, -OH, $-N_3$ oder -F bedeutet;

q und s unabhängig voneinander 1, 2 oder 3 sind;

r 0 oder 1 ist, mit der Maßgabe, daß falls $R^4$ für einen Rest der Formel IV mit $t = 2$ steht, $r = 0$ ist; und

$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;

sowie deren physiologisch verträgliche Salze.

4. Verfahren zur Herstellung einer Verbindung der Formel I, gemäß einem oder mehreren der Ansprüche I bis 3, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

5. Verwendung einer Verbindung der Formel I gemäß einem oder mehreren der Ansprüche 1 bis 3 als Heilmittel.

6. Verwendung einer Verbindung der Formel I gemäß einem oder mehreren der Ansprüche 1 bis 3 bei der Behandlung des Bluthochdrucks.

7. Pharmazeutisches Mittel enthaltend eine Verbindung der Formel I gemäß einem oder mehreren der Ansprüche 1 bis 3.

Patentansprüche für folgende Vertragsstaaten: ES,GR

1. Verfahren zur Herstellung einer Verbindung der Formel I

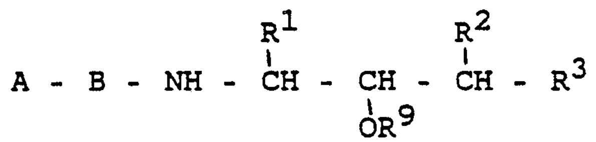 (I),

in welcher
A einen Rest der Formel II

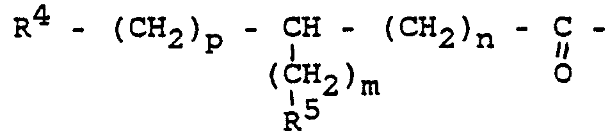 (II)

bedeutet, worin
$R^4$ a) für einen Rest der Formel IV
$R^7$-$S(O)_t$- (IV)
steht, mit
$R^7$ ($C_1$-$C_{10}$)-Alkyl welches durch eine oder mehrere funktionelle Gruppen, beispielsweise Oxo, Hydroxy, ($C_1$-$C_6$)-Alkoxy, Phenyloxy, ($C_1$-$C_6$)-Alkanoyloxy, Halogen, Hydroxysulfonyloxy, Carboxy, ($C_1$-$C_4$)-Alkoxycarbonyl, Carbamoyl, Mono- oder Di-($C_1$-$C_4$)-alkylcarbamoyl, Cyano, Amino, Mono-($C_1$-$C_4$)-alkylamino, Di-($C_1$-$C_4$)-alkylamino, Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom -ist, substituiert sein kann;
($C_2$-$C_8$)-Alkenyl, ($C_2$-$C_8$)-Alkinyl, die beide wie Alkyl substituiert sein können; Hydroxy; ($C_1$-$C_6$)-Alkoxy; ($C_3$-$C_8$)-Cycloalkyl; ($C_5$-$C_{10}$)-Bicycloalkyl; ($C_8$-$C_{10}$)-Tricycloalkyl; ($C_3$-$C_8$)-Cycloalkyl-($C_1$-$C_{10}$)-alkyl; ($C_6$-$C_{14}$)-Aryl, ($C_6$-$C_{14}$)-Aryloxy, ($C_6$-$C_{14}$)-Aryl-($C_1$-$C_{10}$)-alkyl, die im Arylteil gegebenenfalls durch einen oder zwei gleiche oder verschiedene Reste aus der Reihe F, Cl, Br, Hydroxy ($C_1$-$C_7$)-Alkoxy, ($C_1$-$C_7$)-Alkyl, ($C_1$-$C_7$)-Alkoxycarbonyl, Amino oder Trifluormethyl substituiert sind; einen gesättigten aromatischen oder teilaromatischen Heterocyclus, der mono-, bi- oder tricyclisch sein kann und mindestens ein Kohlenstoffatom, ein bis vier Stickstoffatome und/oder ein Schwefel- oder Sauerstoffatom als Ringglieder enthält und durch einen, zwei oder drei gleiche oder verschiedene Reste aus der Reihe F, Cl, Br, Hydroxy, ($C_1$-$C_7$)-Alkoxy, ($C_1$ $C_7$)-Alkyl, ($C_1$-$C_7$)-Alkoxycarbonyl, Amino, Phenyl oder Trifluormethyl substituiert ist; Amino, substituiertes Amino, z.B. Mono-($C_1$-$C_4$)-alkylamino, Di-($C_1$-$C_4$)-alkylamino, Acylamino oder substituiertes Amino, worin die Aminogruppe Teil eines fünf- oder sechsgliedrigen Heterocyclus - enthaltend ein bis zwei Stickstoffatome und gewünschtenfalls ein Sauerstoff- oder Schwefelatom -ist, substituiert sein kann;
und
t 0, 1 oder 2; oder
b) einen gesättigten oder teilweise ungesättigten vier-bis zehngliedrigen monocyclischen oder sieben- bis elfgliedrigen bicyclischen Heterocyclus mit jeweils einem Schwefelatom oder einer SO- oder $SO_2$-Gruppe, die durch einen oder zwei ($C_1$-$C_6$)-Alkyl-, ($C_6$-$C_{14}$)-Aryl-oder ($C_6$-$C_{14}$)-Aryl-($C_1$-$C_6$)-Alkylreste substituiert sein können, wobei jeweils der Arylteil gegebenenfalls wie unter a) beschrieben substituiert sein kann, bedeutet;
$R^5$ Phenyl, 2- oder 3-Thienyl, 2-, 3- oder 4-Pyridyl, 1-, 2- oder 4-Imidazolyl, 1- oder 2-Naphthyl oder 2- oder 3-Benzo[b]thienyl bedeutet, die jeweils gegebenenfalls durch einen, zwei oder drei Reste aus der Gruppe Hydroxy, ($C_1$-$C_4$)-Alkyl, ($C_1$-$C_4$)-Alkoxy, Cl, F, Br, Nitro, Amino, Acetamido, Trifluormethyl oder einen Methylendioxy-Rest substituiert sind;
m 0, 1, 2 oder 3;
n 0, 1 oder 2;
p 0, 1 oder 2 bedeuten;
B einen N terminal mit A und C-terminal mit

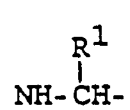

verknüpften Rest einer Aminosäure aus der Reihe Phenylalanin, Histidin, Tyrosin, Tryptophan, Methionin,

Leucin, Isoleucin Asparagin Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-methyl-histidin, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin, 1-, 3-oder 4-Pyrazolylalanin bedeutet;

$R^1$ Wasserstoff, $(C_1\text{-}C_{10})$-Alkyl, $(C_4\text{-}C_7)$-Cycloalkyl, $(C_4\text{-}C_7)$-Cycloalkyl-$(C_1\text{-}C_4)$-alkyl, $(C_6\text{-}C_{14})$-Aryl, $(C_6\text{-}C_{14})$-Aryl-$(C_1\text{-}C_4)$-alkyl oder 1,3-Dithiolan-2-yl-$(C_1\text{-}C_4)$-alkyl bedeutet;

$R^2$ Wasserstoff, $(C_1\text{-}C_{10})$-Alkyl, $(C_6\text{-}C_{14})$-Aryl oder $(C_6\text{-}C_{14})$-Aryl-$(C_1\text{-}C_4)$-alkyl bedeutet;

$R_3^3$ einen Rest der Formel III $-(CH_2)_q - (X)_r - (CH_2)_s - R^6$ (III)

bedeutet, worin

$R^6$ für Wasserstoff, Hydroxy, Amino oder einen fünf-oder sechsgliedrigen monocyclischen oder neun-oder zehngliedrigen bicyclischen Heteroaromaten mit mindestens einem Kohlenstoffatom, einem bis vier Stickstoffatomen und/oder einem Schwefel-oder Sauerstoffatom als Ringglieder steht;

X $-CF_2-$, -CO- oder $-CHR^8$ bedeutet, wobei

$R^8$ für $(C_1\text{-}C_7)$-Alkyl $(C_1\text{-}C_5)$-Alkoxy, $(C_1\text{-}C_5)$-Alkylthio, $(C_1\text{-}C_5)$ Alkylamino, -OH, $-N_3$, -F, -Cl, -Br oder -I steht;

q und s unabhängig voneinander 0, 1, 2, 3 oder 4 bedeuten; und

r 0 oder 1 bedeutet, mit der Maßgabe, daß falls $R^4$ für einen Rest der Formel $R^7\text{-}S(O)_t$- (IV) mit t = 2 steht, r = 0 ist oder n ≠ 0 (Formel II) ist; und

$R^9$ Wasserstoff; $(C_1\text{-}C_{10})$-Alkyl; $(C_1\text{-}C_6)$-Alkanoyl; $(C_6\text{-}C_9)$-Cycloalkanoyl; Phenyl, Phenyl-$(C_1\text{-}C_4)$-alkyl, Benzoyl, die jeweils gegebenenfalls im Aromaten durch einen, zwei oder drei Reste aus der Gruppe $(C_1\text{-}C_4)$-Alkyl, $(C_1\text{-}C_4)$-Alkoxy, Cl, F, Br, Nitro, Trifluormethyl oder ein Methylendioxy substituiert sind; $(C_1\text{-}C_6)$-Alkanoyloxy-$(C_1\text{-}C_2)$-alkyl, $(C_1\text{-}C_6)$-Alkoxycarbonyloxy-$(C_1\text{-}C_2)$-alkyl oder

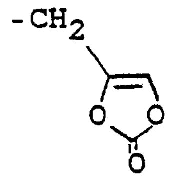

bedeutet,

sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren zur Herstellung einer Verbindung der Formel I, gemäß Anspruch I, in welcher

A einen Rest der Formel II bedeutet, worin

$R^4$ a) für einen Rest der Formel IV steht, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2 Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo-[2.2.1]hept-2-yl, 1-Adamantyl, Cyclopropylmethyl, Cyclobutylmethyl, Cyclopentylmethyl oder Cyclohexylmethyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder a- oder β-Naphthylmethyl, 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4 Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, Methoxy, Ethoxy oder n-Butoxy, Phenoxy, 4-Hydroxyphenoxy oder 3,4-Methylendioxyphenoxy, Amino oder Methylamino, Ethylamino, Isopropylamino, n- oder tert.-Butylamino, Dimethylamino oder Diethylamino, 1-Pyrrolidinyl, 1-Piperidinyl oder 4-Morpholinyl;

und

t 0, 1 oder 2; oder

b) einen gesättigten oder teilweise ungesättigten vier-bis siebengliedrigen monocyclischen oder sieben- bis zehngliedrigen bicyclischen Heterocyclus mit jeweils einem Schwefelatom oder einer SO- oder $SO_2$-Gruppe, wobei der monocyclische Heterocyclus durch ein oder zwei ($C_1$-$C_4$)-Alkyl- oder Phenylreste substituiert sein kann, bedeutet;

$R^5$ Phenyl, 2-Thienyl, 2-Pyridyl, 1-Naphthyl, 2-Benzo[b]thienyl oder 3-Benzo[b]thienyl, die jeweils gegebenenfalls durch einen, zwei oder drei Reste aus der Gruppe Methyl, Ethyl, Isopropyl, tert.-Butyl, Methoxy, Hydroxy, F, Cl, Nitro, Trifluormethyl oder einen Methylendioxyrest substituiert sind, bedeutet;

m 0, 1 oder 2 ist;

n 0 oder 1 ist;

p 0 oder 1 bedeutet;

B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1`-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, 1,3-Dioxolan-2-yl-alanin steht;

$R^1$ Isobutyl, Benzyl oder Cyclohexylmethyl bedeutet;

$R^2$ Wassertoff, Methyl, Isopropyl oder Isobutyl ist;

$R^3$ für einen Rest der Formel III steht; worin

$R^6$ Wasserstoff, Hydroxy, Amino oder einen fünf- oder sechsgliedrigen monocyclischen oder neun- oder zehngliedrigen bicyclischen Heteroaromaten mit mindestens einem Kohlenstoffatom, einem bis vier Stickstoffatomen und/oder einem Schwefel- oder Sauerstoffatom als Ringglieder bedeutet;

X $-CF_2$, -CO- oder $-CHR^8$ ist, wobei

$R^8$ ($C_1$-$C_4$)-Alkyl, ($C_1$-$C_4$)-Alkoxy, -OH, $-N_3$,-F oder -Cl bedeutet;

q und s unabhängig voneinander 0, 1, 2 oder 3 sind;

r 0 oder 1 ist, mit der Maßgabe, daß falls $R^4$ für einen Rest der Formel IV mit $t = 0$ steht, $r = 0$ ist oder $n \neq 0$ (Formel II) ist;

und

$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;

sowie deren physiologisch verträgliche Salze.

3. Verfahren zur Herstellung einer Verbindung der Formel I, gemäß einem oder mehreren der Ansprüche 1 bis 2, in welcher A für einen Rest der Formel II steht, worin

$R^4$ a) einen Rest der Formel IV bedeutet, mit

$R^7$ Methyl, Ethyl, Isopropyl, tert.-Butyl, 2-Hydroxyethyl, 2-Methoxyethyl, 2-Phenoxyethyl, 2-Acetoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, 2-Piperidinoethyl, 2-Benzyloxycarbonylaminoethyl, 2-tert.-Butoxycarbonylaminoethyl, 2-Oxopropyl oder 2-Oxobutyl, Vinyl, Allyl oder 2- oder 3-Butenyl, Ethinyl, 1-Propinyl, oder 2-Propinyl, Cyclopropyl, Cyclobutyl, Cyclopentyl oder Cyclohexyl, Bicyclo-[2.2.1]hept-2-yl, 1-Adamantyl, Cyclopropylmethyl, Cyclobutylmethyl, Cyclopentylmethyl oder Cyclohexylmethyl, Phenyl, 1- oder 2 Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Hydroxy, Methoxy, Ethoxy oder n-Butoxy, Phenoxy, 4-Hydroxyphenoxy oder 3,4-Methylendioxyphenoxy, Amino oder Methylamino, Ethylamino, Isopropylamino, n- oder tert.-Butylamino, Dimethylamino oder 1-Pyrrolidinyl, 1-Piperidinyl oder 4-Morpholinyl; und

t 0, 1 oder 2; oder

b) für Dioxide von Tetrahydrothiophen, Tetrahydrothiopyran, Hexahydrothiepin, Octahydrocyclopenta[b]-thiophen, Octahydrocyclopenta[c]thiophen, Octahydrobenzothiophen oder Octahydroisobenzothiophen steht;

$R^5$ Phenyl, 2-Thienyl, 2-Pyridyl, 1-Naphthyl, die jeweils gegebenenfalls durch Hydroxy, Dihydroxy, Methoxy, Dimethoxy, Ethoxy, Isopropoxy, F, Cl oder Methylendioxy substituiert sind, bedeutet;

m 1 oder 2 ist;
n 0 und
p 0 oder 1 bedeuten;
B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, 1,3-Dioxolan-2-yl-alanin steht;
$R^1$ Isobutyl, Benzyl oder Cyclohexylmethyl bedeutet;
$R^2$ Wasserstoff, Methyl, Isopropyl oder Isobutyl ist;
$R^3$ für einen Rest der Formel III steht, worin
$R^6$ einen fünf- oder sechsgliedrigen monocyclischen oder neun- oder zehngliedrigen bicyclischen Heteroaromaten mit mindestens einem Kohlenstoffatom, einem oder zwei Stickstoffatomen und/oder einem Schwefel- oder Sauerstoffatom als Ringglieder bedeutet; X $-CF_2-$, -CO- oder $-CHR^8$ ist, wobei
$R^8$ Methyl, Ethyl, Isopropyl, Methoxy, Ethoxy, Isopropoxy, -OH, $-N_3$ oder -F bedeutet;
q und s unabhängig voneinander 1, 2 oder 3 sind;
r 0 oder 1 ist, mit der Maßgabe, daß falls $R^4$ für einen Rest der Formel IV mit t = 2 steht, r = 0 ist; und
$R^9$ Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet;
sowie deren physiologisch verträgliche Salze.

4. Verfahren zur Herstellung einer Zubereitung, enthaltend eine Verbindung der Formel I gemäß einem oder mehreren der Ansprüche 1 bis 3, dadurch gekennzeichnet, daß man den Wirkstoff zusammen mit einem physiologisch annehmbaren Träger und gegebenenfalls weiteren Zusatz-, Hilfs- und/oder Konservierungsstoffen in eine geeignete Darreichungsform bringt.